# EUROPÄISCHE PATENTSCHRIFT

(11) **EP 1 235 520 B1**
(45) Veröffentlichungstag und Bekanntmachung des Hinweises auf die Patenterteilung: **03.03.2004**
(21) Anmeldenummer: 99957239.9
(22) Anmeldetag: 10.12.1999
(51) Int. Cl.: A61B 17/02, A61B 17/58

(54) **VORRICHTUNG ZUM DISTRAHIEREN ODER KOMPRIMIEREN VON KNOCHEN ODER KNOCHENTEILEN**
DEVICE FOR DISTRACTING OR COMPRESSING BONES OR BONE FRAGMENTS
DISPOSITIF POUR TENDRE OU COMPRIMER DES OS OU DES PARTIES OSSEUSES

(43) Veröffentlichungstag der Anmeldung: 04.09.2002
(73) Patentinhaber: SYNTHES AG Chur, 7002 Chur (CH)
(72) Erfinder: SCHLÄPFER, Fridolin, CH-8750 Glarus (CH); HESS, Martin, CH-4434 Hölstein (CH)
(74) Vertreter: Lusuardi, Werther Giovanni, Dr.
(86) Internationale Anmeldenummer: PCT/CH1999/000598
(87) Internationale Veröffentlichungsnummer: WO 2001/041652

(56) Entgegenhaltungen:
- DE-A- 4 409 939
- US-A- 3 750 652
- US-A- 4 898 161

## Beschreibung

Die Erfindung bezieht sich auf eine Vorrichtung zur Ausübung von axialen Kräften auf mindestens zwei mit der Vorrichtung verbindbare Knochenfragmente oder Wirbelkörper gemäss dem Oberbegriff des Patentanspruchs 1.

Eine Vorrichtung gemäß dem Oberbegriff von Anspruch 1 ist aus der DE-A-4 409 939 bekannt.

Wird zum Beispiel ein Wirbelkörper krank oder erleidet er einen Defekt, so muss dieser Wirbelkörper unter Umständen aus der Wirbelsäule entfernt werden. Als Ersatz dieses Wirbelkörpers werden dann distanzhaltende Implantate eingesetzt. Solche Implantate umfassen üblicherweise gegeneinander verschiebbare Teile und zwei spezielle Endplatten, welche zur Verankerung des Implantates in den anschliessenden intakten Wirbelkörpern dienen. Andererseits wird oft auch ein internes Fixationssystem so angebracht, dass durch das Fixationssystem zwei Wirbelkörper miteinander verbunden werden, wobei durch das Fixationssystem ein oder sogar mehrere defekte Wirbelkörper überbrückt werden. Bei diesen hauptsächlich plattenförmigen Implantaten werden die endständigen Plattenteile mittels Knochenschrauben an den Wirbelkörpern befestigt. Die Verbindungselemente zwischen diesen endständigen Plattenteilen sind dann teleskopartig, so dass die an die defekten Wirbelkörper angrenzenden Wirbelkörper parallel zur Wirbelsäulenachse distrahierend oder komprimierend bewegt werden können. Wiederum andere Wirbelsäulenfixationssysteme bestehen aus stabförmigen Längsträgern, welche mittels Verbindungsvorrichtungen an Pedikelschrauben befestigbar sind. Diese Verbindungsvorrichtungen sind dabei so gestaltet, dass sie auf den Längsträgern axial verschiebbar sind, wodurch auch eine Distraktion beziehungsweise Kompression der Wirbelkörper möglich ist. Diese Anwendungen erfordern daher häufig eine Spreizung oder auch Kompression von Wirbelkörpern oder Implantatteilen mittels geeigneter Instrumente.

Eine Vorrichtung zur Spreizung von Wirbelkörpern ist aus der US 4,898,161 GRUNDEI bekannt. Diese bekannte Spreizvorrichtung ist zangenartig mit zwei Hebeln und in deren axialer Verlängerung ebenfalls zwei Backen, welche mit Stiften bestückt werden können, ausgebildet. Die Backen enthalten spezielle Führungskulissen und bewegen sich beim Öffnen oder Schliessen der Zange parallel zueinander. Mittels einer schwenkbaren Schrauben-Mutterverbindung zwischen den beiden Hebeln ist die zangenartige Spreizvorrichtung feststellbar. Nachteilig an dieser bekannten Spreizvorrichtung ist, dass keine komprimierenden Verschiebungen möglich sind.

Hier will die Erfindung Abhilfe schaffen. Der Erfindung liegt die Aufgabe zugrunde, eine Vorrichtung zu schaffen, die sowohl eine Distraktion als auch eine Kompression von Knochenfragmenten, Wirbelkörpern oder Implantatteilen ermöglicht und zudem eine sehr präzise und feine Einstellung des Backenabstandes ermöglicht.

Die Erfindung löst die gestellte Aufgabe mit einer Vorrichtung zur Ausübung von axialen Kräften auf mindestens zwei mit der Vorrichtung verbindbare Knochenfragmente oder Wirbelkörper, welche die Merkmale des Anspruchs 1 aufweist.

Die Längsachsen der beiden Hebel kreuzen sich in einem Schnittpunkt und sind mittels eines im Schnittpunkt angeordneten Drehgelenkes drehbar miteinander verbunden. Die Hebel können auch paarweise angeordnet werden, wobei dann die ersten und zweiten Hebel jeweils je einen bezüglich Hülse und Verbindungselemente gegenüberliegenden Hebel umfassen. Andererseits können die ersten und zweiten Hebel jeweils auch auf derselben Seite der Hülse respektive der Verbindungselemente zwei oder mehrere Hebel, welche auch parallel verlaufen können, aufweisen.

Der Längsträger kann in einfacher Weise in der Hülse in axialer Richtung verschiebbar sein, wobei die Betätigung von Längsträger und Hülse durch einfache an diesen Elementen angebrachte Handgriffe oder Hebel erfolgen kann. In einer anderen Ausführungsform können Längsträger und Hülse mittels eines Hebelmechanismus parallel zur Längsachse und relativ zueinander verschiebbar sein. Am Längsträger und and der Hülse sind quer zur Längsachse feste Hebel angebracht, welche mit einer zangenartigen Hilfsvorrichtung aufeinander zu oder voneinander weg bewegbar sind. Die Zangenhebel sind mit Drehgelenken mit den festen Hebeln verbunden und kreuzen sich in einem gemeinsamen Drehgelenk, so dass ein Zusammenpressen der Zangenhebel zu einer aufeinander zu Bewegung der Hebel führt. Zum Auseinanderpressen der Zangenhebel ist zwischen diesen eine Feder angeordnet.

In einer weiteren Ausführungsform der erfindungsgemässen Vorrichtung ist der Längsträger in einem Bereich (B), welcher mit der Lage des vorderen Endstückes an der Hülse korrespondiert, mit einem Aussengewinde versehen, welches zu einem komplementären Innengewinde am vorderen Endstück der Hülse korrespondiert. Zudem ist der Längsträger im ersten Verbindungselement um die Längsachse rotierbar gelagert und bezüglich der Längsachse axial fixiert. Auf diese Weise wird die bezüglich der Längsachse axiale Relativbewegung zwischen Längsträger und Hülse mittels einer Rotationsbewegung des Längsträgers in der Hülse erzeugt.

Andererseits kann der Längsträger in einem Bereich (C) an seinem vorderen Ende, welcher mit dem ersten Verbindungselement verbindbar ist, ein zweites Aussengewinde aufweisen und das erste Verbindungselement mittels eines zum zweiten Aussengewinde komplementären Innengewindes mit dem vorderen Ende des Längsträgers verbunden sein.

Die Gewinde können gleichgängig sein, aber unterschiedliche Gewindesteigungen aufweisen oder gegenläufig sein, wobei dann das eine Aussengewinde ein Rechtsgewinde ist, während das andere Aussengewinde ein Linksgewinde ist. Die korrespondierenden Innengewinde sind mit entsprechend gängigen Gewinden ausgestattet. Ebenfalls möglich sind mehrgängige Gewinde.

Die Hebel können an beiden Verbindungselementen drehbar angeordnet sein. Ist zudem ein Drehpunkt eines Hebels an einem Verbindungselement parallel zur Längsachse des Verbindungselementes verschiebbar, wird eine relative Verschiebung der Verbindungselemente zueinander möglich, wobei die Längsachsen der Verbindungsselemente parallel bleiben und die Verbindungselemente sich entlang einer zu ihren Längsachsen senkrechten Geraden verlaufen.

Der oder die ersten Hebel können auch starr mit dem entsprechenden Verbindungselement verbunden sein, wodurch sich die Verbindungselemente relativ zueinander auf einer ebenen Kurve bewegen.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung ein Spreizen und Komprimieren mit derselben Vorrichtung auch bei grossen Wegen der Spreizung oder Komprimierung durchgeführt werden kann, eine parallele Verschiebung der Knochenfragmente, Wirbelkörper oder Implantatteile gewährleistet wird und zusätzlich ein implantatspezifisches Werkzeug oder Instrument, beispielsweise auch ein Schraubendreher durch die Vorrichtung durchgeführt werden kann.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ansicht einer Ausführungsform der erfindungsgemässen Vorrichtung;
Fig. 2 eine Ansicht einer weiteren Ausführungsform der erfindungsgemässen Vorrichtung;
Fig. 3 wiederum eine weitere Ausführungsform der erfindungsgemässen Vorrichtung; und
Fig. 4 eine zangenartige Betätigungsvorrichtung zum Bedienen einzelner Ausführungsformen der erfindungsgemässen Vorrichtung.

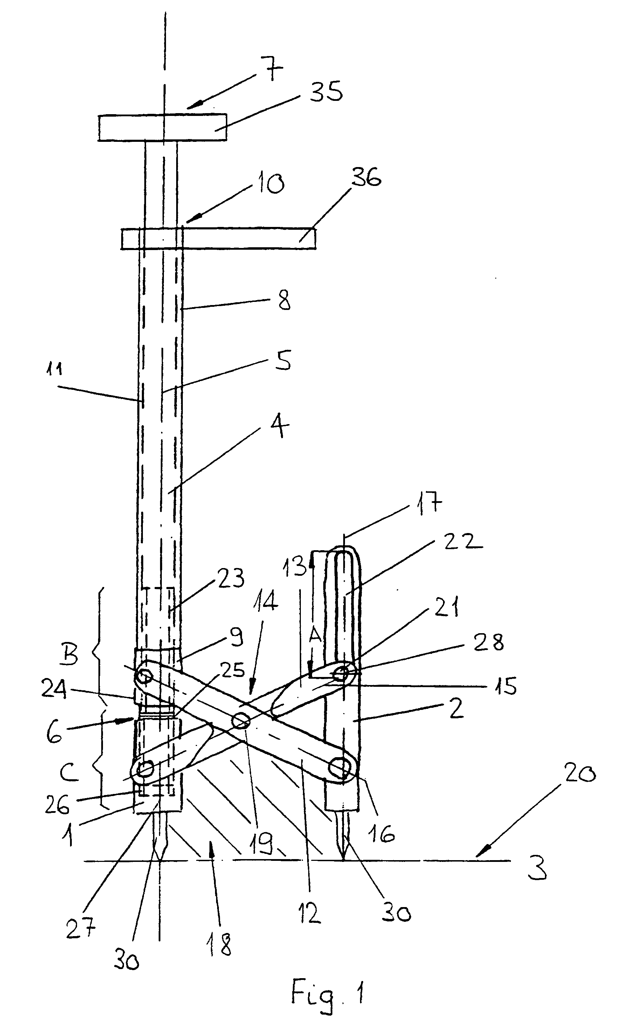

Die in Fig. 1 dargestellte Ausführungsform der erfindungsgemässen Vorrichtung umfasst ein erstes Verbindungselement 1 mit einer Längsachse 27, welches mit einem Knochenfragment oder Wirbelkörper verbindbar ist, sowie ein zweites longitudinales Verbindungselement 2 mit einer Längsachse 17, welches mit einem weiteren Knochenfragment oder Wirbelkörper verbindbar ist. Die Längsachsen 17;27 der Verbindungselement spannen eine Ebene 18 auf. Knochen- oder Implantatseitig sind an den Verbindungselementen 1;2 Spitzen 30 angebracht, welche mit Knochenfragmenten, Wirbelkörpern oder Implantatteilen verbindbar sind und sich bei der Bedienung der Vorrichtung auf einer in der Ebene 18 liegenden, senkrecht zu den Längsachsen 17;27 verlaufenden Geraden 3 bewegen. Zudem ist das zweite Verbindungselement 2 relativ zum ersten Verbindungselement 1 so verschiebbar, dass sich die Längsachse 17 in der Ebene 18 parallel zur Längsachse 27 bewegt. Zur Bedienung der erfindungsgemässen Vorrichtung ist diese ferner mit einem zur Längsachse 27 des ersten Verbindungselementes 1 koaxialen, longitudinalen Längsträger 4 mit einer zur Längsachse 27 konzentrischen Längsachse 5, einem hinteren Ende 7 und einem vorderen, mit dem ersten Verbindungselement 1 verbindbaren Ende 6 ausgestattet. Die Längsachse 5 verläuft bei dieser Ausführungsform in der Ebene 18. Eine zur Längsachse 5 koaxiale rohrartige Hülse 8, welche eine koaxial durchgehende Bohrung 11 aufweist, gestattet eine zur Längsachse 5 koaxiale Verschiebung der Hülse 8 über dem Längsträger 4. Ferner umfasst die Hülse 8 ein vorderes gegen das erste Verbindungselement 1 gerichtetes Endstück 9 und ein hinteres Endstück 10. Die Verschiebung der beiden Verbindungselemente 1;2 relativ zueinander erfolgt über einen ersten, das vordere Endstück 9 und das zweite Verbindungselement 2 verbindenden Hebel 12 mit einer zur Ebene 18 im wesentlichen parallelen Längsachse 16, wobei der Hebel 12 am vorderen Endstück 9 und am zweiten Verbindungselement 2 so drehbar angeordnet, dass der Hebel 12 gegenüber dem vorderen Endstück 9 und dem zweiten Verbindungselement 2 schwenkbar ist, so dass sich die Längsachse 16 parallel zur Ebene 18 bewegt, und einen zweiten Hebel 13 mit einer zur Ebene 18 im wesentlichen parallelen Längsachse 15, wobei der zweite Hebel 13 am ersten Verbindungselement 1 und am zweiten Verbindungselement 2 so drehbar angeordnet ist, dass die Längsachse 15 parallel zur Ebene 18 schwenkbar ist. Die Längsachsen 15;16 der beiden Hebel 12;13 kreuzen sich in einem Schnittpunkt 19 und sind mittels eines im Schnittpunkt 19 angeordneten Drehgelenkes 14 miteinander verbunden. Durch diese Ausführung der scherenartig angeordneten Hebel 12;13 lässt sich erreichen, dass eine Relativbewegung zwischen dem vorderen Endstück 9 und dem ersten Verbindungselement 1 parallel zur Längsachse 5 eine bezüglich der Längsachsen 17 und 27 parallele Relativbewegung zwischen dem ersten und dem zweiten Verbindungselement 1;2 parallel zu der parallel zur Ebene 18 und rechtwinklig zur Längsachse 5 verlaufenden Geraden 3 bewirkt.

Das zweite Verbindungselement 2 enthält eine Führung 22, so dass die Drehung des zweiten Hebels 13 relativ zum zweiten Verbindungselement 2 um die Längsachse 28 eines in der Führung 22 bewegbaren Bolzens 21 ausgeführt wird. Diese Führung 22 verläuft parallel zur Längsachse 17 und weist eine Länge A auf, wodurch der Bolzen 21 parallel zur Längsachse 17 in der Führung 22 verschiebbar ist.

Der Längsträger 4 weist in einem Bereich B, welcher mit der Lage des vorderen Endstückes 9 korrespondiert, ein erstes Aussengewinde 23 auf, während das vordere Endstück 9 ein zum ersten Aussengewinde 23 komplementäres Innengewinde 24 aufweist. Ebenfalls weist der Längsträger 4 in einem Bereich C an seinem vorderen Ende 6, welcher mit dem ersten Verbindungselement 1 verbindbar ist, ein zweites Aussengewinde 25 auf und das erste Verbindungselement 1 ist mittels eines zum zweiten Aussengewinde 25 komplementären Innengewindes 26 mit dem vorderen Ende 6 des Längsträgers 4 verbunden. Die Aussengewinde 23 und 25 auf dem Längsträger 4 weisen eine gegenläufige Gewindesteigung auf. Das Aussengewinde 25 im Bereich C ist als Rechtsgewinde ausgebildet, während das Aussengewinde 23 im Bereich B als Linksgewinde ausgebildet ist. Analog dazu weisen die die Innengewinde 24;26 im vorderen Endstück 9 und im ersten Verbindungselement 1 gegenläufige Gewindesteigungen auf. Damit wird erreicht, dass bei einer Drehung des Längsträgers 4 um die Längsachse 5 das vordere Endstück 9 der Hülse 8 und das erste Verbindungselement 1 je nach Drehrichtung des Längsträgers 4 aufeinander zu bewegt oder voneinander entfernt werden. Durch diese so erreichte Relativbewegung des vorderen Endstückes 9 und des ersten Verbindungselementes 1 wird die Relativbewegung der beiden Verbindungselemente 1;2 relativ zueinander ausgeübt. Zur Ausübung der Drehbewegung zwischen Längsträger 4 und Hülse 8 ist der Längsträger 4 an seinem hinteren Ende 7 mit einem ersten Handgriff 35 und die Hülse 8 ebenfalls an ihrem hinteren Ende 10 mit einem zweiten Handgriff 36 versehen.

Der Längsträger 4 und das erste Verbindungselement 1 können koaxial zu deren Längsachsen 5;27 durchbohrt sein, so dass ein Schraubendreher durch diese Bohrungen durchführbar ist. Ebenfalls kann das zweite Verbindungselement 2 koaxial zu seiner Längsachse 17 durchbohrt sein, so dass ein Schraubendreher durch diese Bohrung durchführbar ist.

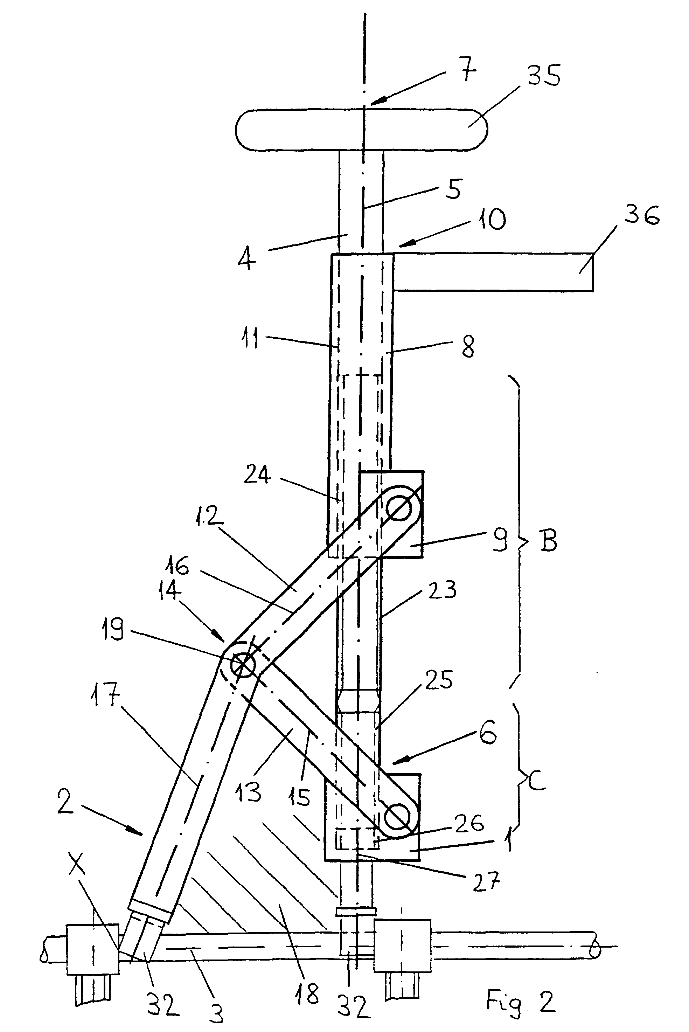

Die in Fig. 2 dargestellte Ausführungsform der erfindungsgemässen Vorrichtung unterscheidet sich von der in Fig. 1 gezeigten Ausführungsform lediglich darin, dass sich die Längsachsen 15;16 der beiden Hebel 12;13 an dem vom vorderen Endstück 9 respektive vom ersten Verbindungselement 1 entfernten Ende der Hebel 12;13 schneiden. Das Drehgelenk 14, welches die beiden Hebel 12;13 verbindet ist daher auch an diesen Enden angebracht. Die Hebel 12;13 sind im wesentlichen gleich lang und das zweite Verbindungselement 2 ist starr mit dem ersten Hebel 12 verbunden, wobei sich die Längsachse 16 des Hebels 12 und die Längsachse 17 des zweiten Verbindungselementes 2 unter einem Winkel schneiden. Bei dieser Ausführungsform der erfindungsgemässen Vorrichtung bewegen sich bei Bedienung der Vorrichtung die Verbindungselemente 1;2 nicht mehr so, dass deren Längsachsen 17;27 sich parallel bewegen. Die Verbindungselemente 1;2 bewegen sich hier auf einer in der Ebene 18 liegenden Kurve. Allerdings lässt sich auf dem zweiten Verbindungselement 2 ein Punkt X definieren, welcher sich im wesentlichen auf einer zur Ebene 18 parallelen Geraden 3 bewegt. An den Verbindungselementen 1;2 sind U-förmige Platten 32 angebracht sind, welche an Implantatteilen zur Anlage bringbar sind.

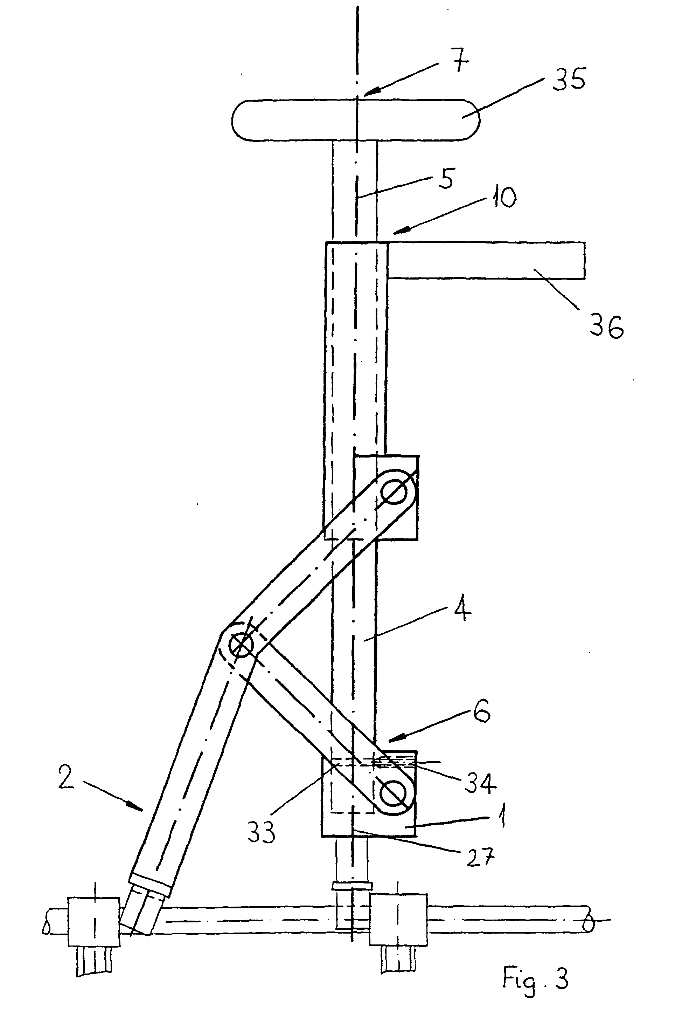

Die in Fig. 3 dargestellte Ausführungsform der erfindungsgemässen Vorrichtung unterscheidet sich von der in Fig. 2 gezeigten Ausführungsform nur darin, dass der Längsträger 4 parallel zur Längsachse 5 in der Hülse 8 verschiebbar ist und an seinem vorderen Ende 6 innerhalb des ersten Verbindungselementes 1 bezüglich der Längsachse 27 axialfest aber um die Längsachse 27 drehbar angeordnet ist. Auf diese Weise wird erreicht, dass die Relativbewegung zwischen Längsträger 4 und Hülse 8 eine reine axiale Verschiebung ist, welche durch aufeinander zu bewegen oder voneinander weg bewegen der Handgriffe 35 und 36 erreichbar ist. Die rotierbare aber axialfeste Lagerung des Längsträgers 4 im ersten Verbindungselement 1 erfolgt durch eine Stiftschraube 34, welche in das erste Verbindungselement 1 eingeschraubt ist und in eine dazu korrespondierende Rille 33 am Längsträger 4 ragt.

Der in Fig. 3 gezeigte Bewegungsmechanismus zwischen Längsträger 4 und Hülse 8 kann ohne weiteres auch auf die in Fig. 1 gezeigte Ausführungsform der erfindungsgemässen Vorrichtung übertragen werden.

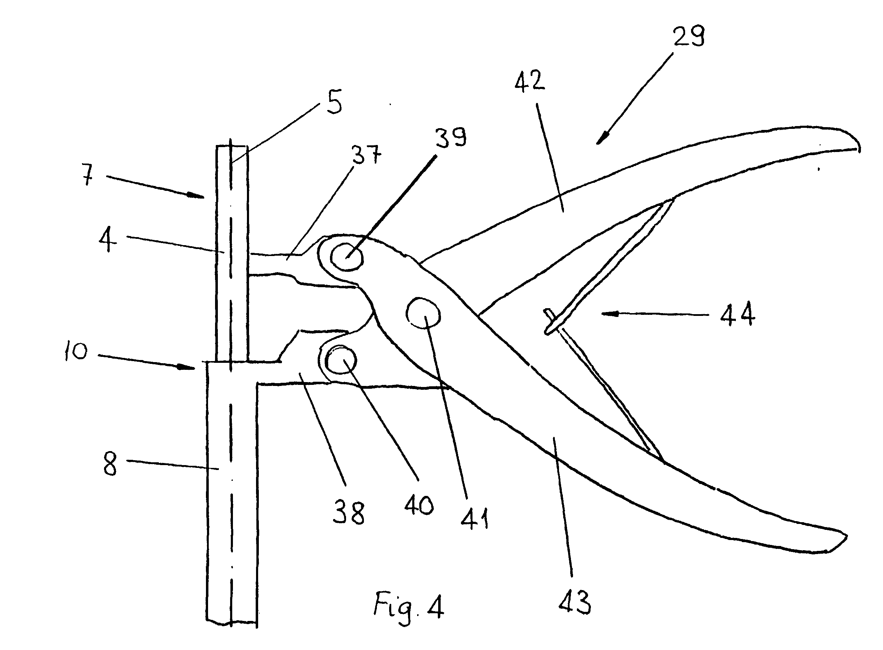

Fig. 4 zeigt einen Ausschnitt aus einer Ausführungsform der erfindungsgemässen Vorrichtung, beipielsweise der Ausführungsform gemäss Fig. 3, bei welcher der Längsträger 4 und die Hülse 8 mittels eines Hebelmechanismus 29 parallel zur Längsachse 5 und relativ zueinander verschiebbar sind. An den Enden 7 und 10 des Längsträgers 4 und der Hülse 8 sind quer zur Längsachse 5 feste Hebel 37 und 38 angebracht, welche mit einer Zange aufeinander zu oder voneinander weg bewegbar sind. Die Zangenhebel 42 und 43 sind mit Drehgelenken 39 und 40 mit den festen Hebeln 37 und 38 verbunden und kreuzen sich in einem gemeinsamen Drehgelenk 41, so dass ein Zusammenpressen der Zangenhebel 42 und 43 zu einer aufeinander zu Bewegung der Hebel 37 und 38 führt. Zum Auseinanderpressen der Zangenhebel 42 und 43 ist zwischen diesen eine Feder 44 angeordnet.

## Patentansprüche

1. Vorrichtung zur Ausübung von Distraktions- oder Kompressions-Kräften auf mindestens zwei mit der Vorrichtung verbindbare Knochenfragmente oder Wirbelkörper, mit
A) einem ersten Verbindungselement (1) mit einer Längsachse (27), welches mit einem Knochenfragment oder Wirbelkörper verbindbar ist; und
B) einem zweiten longitudinalen Verbindungselement (2) mit einer Längsachse (17), welches mit einem weiteren Knochenfragment oder Wirbelkörper verbindbar ist, wobei die Längsachsen (17;27) eine Ebene (18) aufspannen und das zweite Verbindungselement (2) relativ zum ersten Verbindungselement (1) so verschiebbar ist, dass sich seine Längsachse (17) in der Ebene (18) bewegt,
**dadurch gekennzeichnet, dass** die Vorrichtung ferner
C) einen zur Längsachse (27) des ersten Verbindungselementes koaxialen, longitudinalen Längsträger (4) mit einer Längsachse (5), einem hinteren Ende (7) und einem vorderen, mit dem ersten Verbindungselement (1) verbindbaren Ende (6) umfasst, wobei die Längsachse (5) des Längsträgers (4) parallel zur Ebene (18) verläuft;
D) eine zum Längsträger (4) koaxiale rohrartige Hülse (8), welche eine koaxial durchgehende Bohrung (11) aufweist, worin der Längsträger (4) parallel zu seiner Längsachse (5) verschiebbar ist, und welche ein vorderes gegen das erste Verbindungselement (1) gerichtetes Endstück (9) und ein hinteres Endstück (10) umfasst;
E) mindestens einen ersten, das vordere Endstück (9) und das zweite Verbindungselement (2) verbindenden Hebel (12) mit einer zur Ebene (18) im wesentlichen parallelen Längsachse (16), welcher mindestens am vorderen Endstück (9) so drehbar angeordnet ist, dass die Längsachse (16) im wesentlichen parallel zur Ebene (18) schwenkbar ist; und
F) mindestens einen zweiten Hebel (13) mit einer zur Ebene (18) im wesentlichen parallelen Längsachse (15) , welcher am ersten Verbindungselement (1) und am zweiten Verbindungselement (2) so drehbar angeordnet ist, dass die Längsachse (15) im wesentlichen parallel zur Ebene (18) schwenkbar ist, umfasst; so dass
G) eine Relativbewegung zwischen dem vorderen Endstück (9) und dem ersten Verbindungselement (1) parallel zur Längsachse (5) eine Relativbewegung zwischen dem ersten und dem zweiten Verbindungselement (1;2) entlang einer parallel zur Ebene (18) und im wesentlichen quer zur Längsachse (5) verlaufenden Linie (20) bewirkt.

2. Vorrichtung nach Anspruch 1, **dadurch gekennzeichnet, dass** parallel zur Ebene (18) im Spezialfall in der Ebene (18) bedeutet.

3. Vorrichtung nach Anspruch 1 oder 2, **dadurch gekennzeichnet, dass** die Linie (20) eine Gerade (3) ist.

4. Vorrichtung nach einem der Ansprüche 1 bis 3, **dadurch gekennzeichnet, dass** auf dem zweiten Verbindungselement (2) ein Punkt (X) definierbar ist, welcher sich bei der Relativbewegung zwischen dem vorderen Endstück (9) und dem ersten Verbindungselement (1) im wesentlichen auf der parallel zur Ebene (18) verlaufenden Geraden (3) bewegt.

5. Vorrichtung nach Anspruch 3 oder 4, **dadurch gekennzeichnet, dass** die Gerade (3) senkrecht auf Längsachse (5) steht.

6. Vorrichtung nach einem der Ansprüche 1 bis 5, **dadurch gekennzeichnet, dass** sich die Längsachsen (15;16) in einem Schnittpunkt (19) kreuzen.

7. Vorrichtung nach Anspruch 6, **dadurch gekennzeichnet, dass** die Hebel (12;13) mittels eines im Schnittpunkt (19) angeordneten Drehgelenkes (14) miteinander verbunden sind.

8. Vorrichtung nach einem der Ansprüche 1 bis 7, **dadurch gekennzeichnet, dass** die Hebel (12;13) im wesentlichen gleich lang sind.

9. Vorrichtung nach einem der Ansprüche 1 bis 8, **dadurch gekennzeichnet, dass** das zweite Verbindungselement (2) im wesentlichen parallel relativ zum ersten Verbindungselement (1) verschiebbar ist.

10. Vorrichtung Anspruch 9, **dadurch gekennzeichnet, dass** das zweite Verbindungselement (2) im wesentlichen parallel zur Längsachse (5) relativ zum ersten Verbindungselement (1) verschiebbar ist.

11. Vorrichtung nach einem der Ansprüche 1 bis 10, **dadurch gekennzeichnet, dass** der erste Hebel (12) am zweiten Verbindungselement (2) so drehbar angeordnet ist, dass die Längsachse (16) im wesentlichen parallel zur Ebene (18) schwenkbar ist.

12. Vorrichtung nach einem der Ansprüche 1 bis 11, **dadurch gekennzeichnet, dass** das zweite Verbindungselement (2) eine Führung (22) umfasst und die Drehung des zweiten Hebels (13) relativ zum zweiten Verbindungselement (2) um die Längsachse (28) eines in der Führung (22) bewegbaren Bolzens (21) ausgeführt wird.

13. Vorrichtung nach Anspruch 12, **dadurch gekennzeichnet, dass** die Führung (22) parallel zur Längsachse (17) eine Länge (A) aufweist, wodurch der Bolzen (21) parallel zur Längsachse (17) in der Führung (22) verschiebbar ist.

14. Vorrichtung nach einem der Ansprüche 1 bis 8, **dadurch gekennzeichnet, dass** das zweite Verbindungselement (2) und der erste Hebel (12) starr miteinander verbunden sind.

15. Vorrichtung nach Anspruch 14, **dadurch gekennzeichnet, dass** die Hebel (12;13) ein gleichschenkliges Dreieck bilden.

16. Vorrichtung nach einem der Ansprüche 1 bis 15, **dadurch gekennzeichnet, dass**
A) der Längsträger (4) in einem Bereich (B), welcher mit der Lage des vorderen Endstückes (9) korrespondiert, ein Aussengewinde (23) aufweist;
B) das vordere Endstück (9) ein zum Aussengewinde (23) komplementäres Innengewinde (24) aufweist;
C) der Längsträger (4) im ersten Verbindungselement (1) um die Längsachse (5) rotierbar gelagert ist; und
D) der Längsträger (4) im ersten Verbindungselement (1) bezüglich der Längsachse (5) axial fixiert ist.

17. Vorrichtung nach einem der Ansprüche 1 bis 15, **dadurch gekennzeichnet, dass**
A) der Längsträger (4) in einem Bereich (B), welcher mit der Lage des vorderen Endstückes (9) korrespondiert, ein erstes Aussengewinde (23) aufweist;
B) das vordere Endstück (9) ein zum ersten Aussengewinde (23) komplementäres Innengewinde (24) aufweist;
C) der Längsträger (4) in einem Bereich (C) an seinem vorderen Ende (6), welcher mit dem ersten Verbindungselement (1) verbindbar ist, ein zweites Aussengewinde (25) aufweist; und
D) das erste Verbindungselement (1) mittels eines zum zweiten Aussengewinde (25) komplementären Innengewindes (26) mit dem vorderen Ende (6) des Längsträgers (4) verbunden ist.

18. Vorrichtung nach Anspruch 17, **dadurch gekennzeichnet, dass** die Aussengewinde (23;25) gleichgängig sind, aber unterschiedliche Gewindesteigungen aufweisen.

19. Vorrichtung nach Anspruch 17, **dadurch gekennzeichnet, dass** die Aussengewinde (23;25) gegenläufig sind.

20. Vorrichtung nach Anspruch 19, **dadurch gekennzeichnet, dass** das Aussengewinde (25) im Bereich (C) ein Rechtsgewinde ist.

21. Vorrichtung nach Anspruch 19 oder 20, **dadurch gekennzeichnet, dass** das Aussengewinde (23) im Bereich (B) ein Linksgewinde ist.

22. Vorrichtung nach einem der Ansprüche 17 bis 21, **dadurch gekennzeichnet, dass** das Aussengewinde (23) im Bereich (B) mehrgängig ist.

23. Vorrichtung nach einem der Ansprüche 17 bis 21, **dadurch gekennzeichnet, dass** das Aussengewinde (25) im Bereich (C) mehrgängig ist.

24. Vorrichtung nach einem der Ansprüche 1 bis 16, **dadurch gekennzeichnet, dass** der Längsträger (4) im ersten Verbindungselement (1) mindestens axial bezüglich der Längsachse (5) fixiert ist.

25. Vorrichtung nach einem der Ansprüche 1 bis 16 und 24, **dadurch gekennzeichnet, dass** der Längsträger (4) und die Hülse (8) mittels eines Hebelmechanismus (29) parallel zur Längsachse (5) und relativ zueinander verschiebbar sind.

26. Vorrichtung nach Anspruch 25, **dadurch gekennzeichnet, dass** der Hebelmechanismus (29) zangenartig mit einer Feder (44) zur elastischen Spreizung des Hebelmechanismus (29) ausgestaltet ist.

27. Vorrichtung nach einem der Ansprüche 1 bis 26, **dadurch gekennzeichnet, dass** der Längsträger (4) und das erste Verbindungselement (1) koaxial zu deren Längsachsen (5;27) durchbohrt sind, so dass ein Schraubendreher durch diese Bohrungen durchführbar ist.

28. Vorrichtung nach einem der Ansprüche 1 bis 27, **dadurch gekennzeichnet, dass** das zweite Verbindungselement (2) koaxial zu seiner Längsachse (17) durchbohrt ist, so dass ein Schraubendreher durch diese Bohrung durchführbar ist.

29. Vorrichtung nach einem der Ansprüche 1 bis 28, **dadurch gekennzeichnet, dass** an den Verbindungselementen (1;2) Spitzen (30) angebracht sind, welche mit Knochenfragmenten, Wirbelkörpern oder Implantatteilen verbindbar sind.

30. Vorrichtung nach einem der Ansprüche 1 bis 28, **dadurch gekennzeichnet, dass** an den Verbindungselementen (1;2) Klingen (31) angebracht sind, welche an Knochenfragmenten, Wirbelkörpern oder Implantatteilen zur Anlage bringbar sind.

31. Vorrichtung nach einem der Ansprüche 1 bis 28, **dadurch gekennzeichnet, dass** an den Verbindungselementen (1;2) U-förmige Platten (32) angebracht sind, welche an Implantatteilen zur Anlage bringbar sind.

32. Vorrichtung nach einem der Ansprüche 1 bis 28, **dadurch gekennzeichnet, dass**
A) die Verbindung zwischen den Verbindungselementen (1;2) und den mit den Wirbelkörpern oder Knochenfragmenten fest verbundenen Implantaten, wie zum Beispiel Knochenschrauben, kugelgelenkartig ausgebildet sind; und
B) die kugelgelenkartige Verbindung reversibel winkelstabil blockierbar ist.

## Claims

1. An apparatus for applying distraction or compression forces on at least two bone fragments or vertebral bodies connectable with said apparatus, including
A) a first connecting member (1) with a longitudinal axis (27) which is connectable to a bone fragment or a vertebral body; and
B) a second, longitudinal connecting member (2) with a longitudinal axis (17) which is connectable to another bone fragment or vertebral body, the longitudinal axes (17;27) forming a plane (18) between them and the second connecting member (2) being displaceable relative to the first connecting member (1) in such a way that its longitudinal axis (17) will move in said plane (18);
**characterised in that** the apparatus further comprises
C) a longitudinal carrier (4) extending coaxially to the longitudinal axis (27) of the first connecting member (1) and including a longitudinal axis (5), a rear end portion (7), as well as a front end portion (6) connectable with the first connecting member (1), the longitudinal axis (5) of the longitudinal carrier (4) extending parallel to said plane (18);
D) a tubular sleeve (8) extending coaxially to the longitudinal carrier (4) and including a coaxial through bore (11) in which the longitudinal carrier (4) is displaceable parallel to its longitudinal axis (5), and which comprises a front end portion (9) facing the first connecting member (1), and a rear end portion (10);
E) at least one first lever (12) connecting the front end portion (9) to the second connecting member (2) and having a longitudinal axis (16) extending substantially parallel to said plane (18), rotatably connected at least to the front end portion (9) in such a way that said longitudinal axis (16) may pivot substantially parallel to said plane (18); and
F) at least one second lever (13) with a longitudinal axis (15) extending substantially parallel to said plane (18), rotatably connected to the first connecting member (1) and the second connecting member (2) in such a way that the longitudinal axis (15) may pivot substantially parallel to said plane (18); so that
G) a relative movement between the front end portion (9) and the first connecting member (1) parallel to said longitudinal axis (5) results in a relative movement between the first and the second connecting members (1;2) along a line (20) extending parallel to said plane (18) and substantially vertically to said longitudinal axis (5).

2. An apparatus as claimed in claim 1, **characterised in that** in the special case the expression 'parallel to the plane (18)' is to be understood as meaning 'coplanar to the plane (18)'.

3. An apparatus as claimed in claim 1 or 2, **characterised in that** the line (20) is a straight line (3).

4. An apparatus as claimed in any of the claims 1 to 3, **characterised in that** a point (X) may be defined on the second connecting member (2) which moves substantially along the straight line (3) extending parallel to the plane (18) as the front end portion (9) and the first connecting member (1) are displaced relative to each other.

5. An apparatus as claimed in claim 3 or 4, **characterised in that** the straight line (3) extends vertically to the longitudinal axis (5).

6. An apparatus as claimed in any of the claims 1 to 5, **characterised in that** the longitudinal axes (15;16) cross at a point of intersection (19).

7. An apparatus as claimed in claim 6, **characterised in that** the levers (12;13) are connected with each other by means of a swivel joint (14) arranged at their point of intersection (19).

8. An apparatus as claimed in any of the claims 1 to 7, **characterised in that** the levers (12;13) are substantially of the same length.

9. An apparatus as claimed in any of the claims 1 to 8, **characterised in that** the second connecting member (2) is displaceable substantially parallel relative to the first connecting member (1).

10. An apparatus as claimed in claim 9, **characterised in that** the second connecting member (2) is displaceable substantially parallel to the longitudinal axis (5) relative to the first connecting member (1).

11. An apparatus as claimed in any of the claims 1 to 10, **characterised in that** the first lever (12) is rotatably connected to the second connecting member (2) in such a way that the longitudinal axis (16) may pivot substantially parallel to the plane (18).

12. An apparatus as claimed in any of the claims 1 to 11, **characterised in that** the second connecting member (2) comprises a guide (22) assuring that the rotation of the second lever (13) relative to the second connecting member (2) is executed about the longitudinal axis (28) of a bolt (21) which is freely movable along the guide (22).

13. An apparatus as claimed in claim 12, **characterised in that** the guide (22) has a length (A) extending parallel to the longitudinal axis (17) and enabling a displacement of the bolt (21) in the guide (22) parallel to the longitudinal axis (17).

14. An apparatus as claimed in any of the claims 1 to 8, **characterised in that** the second connecting member (2) and the first lever (12) are rigidly connected to each other.

15. An apparatus as claimed in claim 14, **characterised in that** the levers (12;13) form an isosceles triangle.

16. An apparatus as claimed in any of the claims 1 to 15, **characterised in that**
A) the longitudinal carrier (4) is provided in an area (B) corresponding to the position of the front end portion (9) with an external screw thread (23);
B) the front end portion (9) has an internal screw thread (24) which is complementary to said external screw thread (23);
C) the longitudinal carrier (4) is mounted within the first connecting member (1) in such a way as to be rotatable about the longitudinal axis (5); and
D) the longitudinal carrier (4) is mounted within the first connecting member (1) in such a way as to be axially fixed relative to the longitudinal axis (5).

17. An apparatus as claimed in any of the claims 1 to 15, **characterised in that**
A) the longitudinal carrier (4) is provided in an area (B) corresponding to the position of the front end portion (9) with a first external screw thread (23);
B) the front end portion (9) has an internal screw thread (24) which is complementary to said external screw thread (23);
C) the longitudinal carrier (4) is provided in an area (C), which is situated on its front end portion (6) and is connectable to the first connecting member (1), with a second external screw thread (25); and
D) the first connecting member (1) is connected to the front end portion (6) of the longitudinal carrier (4) by means of an internal screw thread (26) which is complementary to said second external screw thread (25).

18. An apparatus as claimed in claim 17, **characterised in that** the external screw threads (23;25) are of equal turn but have different pitches.

19. An apparatus as claimed in claim 17, **characterised in that** the external screw threads (23;25) have opposed turns.

20. An apparatus as claimed in claim 19, **characterised in that** the external screw thread (25) in the area (C) is a right-hand thread.

21. An apparatus as claimed in claim 19 or 20, **characterised in that** the external screw thread (23) in the area (B) is a left-hand thread.

22. An apparatus as claimed in any of the claims 17 to 21, **characterised in that** the external screw thread (23) in the area (B) is a multiple thread.

23. An apparatus as claimed in any of the claims 17 to 21, **characterised in that** the external screw thread (25) in the area (C) is a multiple thread.

24. An apparatus as claimed in any of the claims 1 to 16, **characterised in that** the longitudinal carrier (4) is mounted within the first connecting member (1) in such a way as to be at least axially fixed relative to the longitudinal axis (5).

25. An apparatus as claimed in any of the claims 1 to 16, and 24, **characterised in that** the longitudinal carrier (4) and the sleeve (8) are displaceable parallel to the longitudinal axis (5) and relative to each other by means of a lever appliance (29).

26. An apparatus as claimed in claim 25, **characterised in that** the lever appliance (29) is shaped in the form of a forceps and equipped with a spring (44) for flexibly spreading apart said lever appliance (29).

27. An apparatus as claimed in any of the claims 1 to 26, **characterised in that** both the longitudinal carrier (4) and the first connecting member (1) have a through bore extending coaxially to their longitudinal axes (5;27), so that a screw driver may be introduced through these bores.

28. An apparatus as claimed in any of the claims 1 to 27, **characterised in that** the second connecting member (2) has a through bore extending coaxially to its longitudinal axis (17), so that a screw driver may be introduced through this bore.

29. An apparatus as claimed in any of the claims 1 to 28, **characterised in that** the connecting members (1;2) are equipped with pins (30) which are connectable to bone fragments, vertebral bodies or implant parts.

30. An apparatus as claimed in any of the claims 1 to 28, **characterised in that** the connecting members (1;2) are equipped with blades (31) which are attachable to bone fragments, vertebral bodies or implant parts.

31. An apparatus as claimed in any of the claims 1 to 28, **characterised in that** the connecting members (1;2) are equipped with U-shaped plates (32) which are attachable to implant parts.

32. An apparatus as claimed in any of the claims 1 to 28, **characterised in that**
A) the connection between the connecting members (1;2) and the implants such as bone screws, which are fixedly connected to the vertebral bodies or bone fragments, is realised in the form of a ball-and-socket joint; and
B) the ball joint-like connection is reversibly blockable in an angularly stable manner.

## Revendications

1. Dispositif pour exercer des forces d'écartement ou de compression sur au moins deux fragments d'os ou corps de vertèbre aptes à être reliés au dispositif, avec:
A) un premier élément de liaison (1) qui présente un axe longitudinal (27) et qui peut être relié à un fragment d'os ou un corps de vertèbre et
B) un deuxième élément longitudinal de liaison (2) qui présente un axe longitudinal (17) et qui peut être relié à un autre fragment d'os ou un autre corps de vertèbre, les axes longitudinaux (17; 27) sous-tendant un plan (18) et le deuxième élément de liaison (2) pouvant être déplacé par rapport au premier élément de liaison (1) de telle sorte que son axe longitudinal (17) se déplace dans le plan (18),
**caractérisé en ce que** le dispositif présente en outre :
C) un support longitudinal (4) coaxial par rapport à l'axe longitudinal (27) du premier élément de liaison et qui présente un axe longitudinal (5), une extrémité arrière (7) et une extrémité avant (6) qui peut être reliée au premier élément de liaison (1), l'axe longitudinal (5) du support longitudinal (4) s'étendant parallèlement au plan (18),
D) une douille tubulaire (8) coaxiale par rapport au support longitudinal (4) qui est traversée par un alésage (11) coaxial dans lequel le support longitudinal (4) peut coulisser parallèlement à son axe longitudinal (5) et qui comprend une pièce d'extrémité avant (9) dirigée vers le premier élément de liaison (1) et une pièce d'extrémité arrière (10),
E) au moins un premier levier (12) qui relie la pièce d'extrémité avant (9) et le deuxième élément de liaison (2), dont l'axe longitudinal (16) est essentiellement parallèle au plan (18) et qui est disposé à rotation au moins sur la pièce d'extrémité avant (9) de telle sorte que son axe longitudinal (16) peut pivoter de manière essentiellement parallèle au plan (18) et
F) au moins un deuxième levier (13) dont l'axe longitudinal (15) est essentiellement parallèle au plan (18) et qui est disposé à rotation sur le premier élément de liaison (1) et sur le deuxième élément de liaison (2) de telle sorte que son axe longitudinal (15) peut pivoter de manière essentiellement parallèle au plan (18), de sorte que
G) un déplacement relatif entre la pièce d'extrémité avant (9) et le premier élément de liaison (1) parallèlement à l'axe longitudinal (5) a pour effet un déplacement relatif entre le premier et le deuxième élément de liaison (1; 2) le long d'une ligne (20) parallèle au plan (18) et essentiellement transversale par rapport à l'axe longitudinal (5).

2. Dispositif selon la revendication 1, **caractérisé en ce que** dans un cas spécial, l'expression "parallèle au plan (18)" signifie "dans le plan (18)".

3. Dispositif selon la revendication 1 ou 2, **caractérisé en ce que** la ligne (20) est une droite (3).

4. Dispositif selon l'une des revendications 1 à 3, **caractérisé en ce que** sur le deuxième élément de liaison (2) peut être défini un point (X) qui se déplace essentiellement sur la droite (3) parallèle au plan (18) lors d'un déplacement relatif entre la pièce d'extrémité avant (9) et le premier élément de liaison (1).

5. Dispositif selon les revendications 3 ou 4, **caractérisé en ce que** la droite (3) est perpendiculaire à l'axe longitudinal (5).

6. Dispositif selon l'une des revendications 1 à 5, **caractérisé en ce que** les axes longitudinaux (15; 16) se croisent en un point d'intersection (19).

7. Dispositif selon la revendication 6, **caractérisé en ce que** les leviers (12; 13) sont reliés l'un à l'autre au moyen d'une articulation de rotation (14) disposée au point d'intersection (19).

8. Dispositif selon l'une des revendications 1 à 7, **caractérisé en ce que** les leviers (12; 13) présentent essentiellement la même longueur.

9. Dispositif selon l'une des revendications 1 à 8, **caractérisé en ce que** le deuxième élément de liaison (2) peut coulisser de manière essentiellement parallèle au premier élément de liaison (1).

10. Dispositif selon la revendication 9, **caractérisé en ce que** le deuxième élément de liaison (2) peut coulisser par rapport au premier élément de liaison (1) de manière essentiellement parallèle à l'axe longitudinal (5).

11. Dispositif selon l'une des revendications 1 à 10, **caractérisé en ce que** le premier levier (12) est disposé à rotation sur le deuxième élément de liaison (2) de telle sorte que l'axe longitudinal (16) peut pivoter de manière essentiellement parallèle au plan (18).

12. Dispositif selon l'une des revendications 1 à 11, **caractérisé en ce que** le deuxième élément de liaison (2) comprend un guide (22) et **en ce que** la rotation du deuxième levier (13) par rapport au deuxième élément de liaison (2) est réalisée autour de l'axe longitudinal (28) d'un boulon (21) apte à se déplacer dans le guide (22).

13. Dispositif selon la revendication 12, **caractérisé en ce que** parallèlement à l'axe longitudinal (17), le guide (22) présente une longueur (A) qui permet au boulon (21) de se déplacer dans le guide (22) parallèlement à l'axe longitudinal (17).

14. Dispositif selon l'une des revendications 1 à 8, **caractérisé en ce que** le deuxième élément de liaison (2) et le premier levier (12) sont reliés l'un à l'autre rigidement.

15. Dispositif selon la revendication 14, **caractérisé en ce que** les leviers (12; 13) forment un triangle équilatéral.

16. Dispositif selon l'une des revendications 1 à 15, **caractérisé en ce que**
A) dans une zone (B) qui correspond à la position de la pièce d'extrémité avant (9), le support longitudinal (4) présente un filet extérieur (23),
B) la pièce d'extrémité avant (9) présente un filet intérieur (24) complémentaire du filet extérieur (23),
C) le support longitudinal (4) est monté dans le premier élément de liaison (1) à rotation autour de l'axe longitudinal (5) et
D) le support longitudinal (4) est immobilisé dans le premier élément de liaison (1) axialement par rapport à l'axe longitudinal (5).

17. Dispositif selon l'une des revendications 1 à 15, **caractérisé en ce que**
A) dans une zone (B) qui correspond à la position de la pièce d'extrémité avant (9), le support longitudinal (4) présente un premier filet extérieur (23),
B) la pièce d'extrémité avant (9) présente un filet intérieur (24) complémentaire du premier filet extérieur (23),
C) dans une zone (C) de son extrémité avant (6) qui peut être reliée au premier élément de liaison (1), le support longitudinal (4) présente un deuxième filet extérieur (25) et
D) le premier élément de liaison (1) est relié à l'extrémité avant (6) du support longitudinal (4) au moyen d'un filet intérieur (26) complémentaire du deuxième filet extérieur (25).

18. Dispositif selon la revendication 17, **caractérisé en ce que** les filets extérieurs (23; 25) sont dans le même sens mais ont des pas différents.

19. Dispositif selon la revendication 17, **caractérisé en ce que** les filets extérieurs (23, 25) ont des sens opposés.

20. Dispositif selon la revendication 19, **caractérisé en ce que** dans la zone (C), le filet extérieur (25) est un filet droit.

21. Dispositif selon les revendications 19 ou 20, **caractérisé en ce que** dans la zone (B), le filet extérieur (23) est un filet gauche.

22. Dispositif selon l'une ou plusieurs des revendications 17 à 21, **caractérisé en ce que** dans la zone (B), le filet extérieur (23) présente plusieurs pas.

23. Dispositif selon l'une des revendications 17 à 21, **caractérisé en ce que** dans la zone (C), le filet extérieur (25) présente plusieurs pas.

24. Dispositif selon l'une des revendications 1 à 16, **caractérisé en ce que** le support longitudinal (4) est immobilisé dans le premier élément de liaison (1) au moins dans le sens axial par rapport à l'axe longitudinal (5).

25. Dispositif selon l'une des revendications 1 à 16 et la revendication 24, **caractérisé en ce que** le support longitudinal (4) et la douille (8) peuvent être déplacés parallèlement à l'axe longitudinal (5) et l'un par rapport à l'autre au moyen d'un mécanisme à levier (29).

26. Dispositif selon la revendication 25, **caractérisé en ce que** le mécanisme à levier (29) est configuré en forme de pince avec un ressort (44) qui permet l'écartement élastique du mécanisme de levier (29).

27. Dispositif selon l'une des revendications 1 à 26, **caractérisé en ce que** le premier support longitudinal (4) et le premier élément de liaison (1) sont traversés par un alésage coaxial par rapport à leur axe longitudinal (5; 27) de telle sorte qu'un tournevis peut être passé dans ces alésages.

28. Dispositif selon l'une des revendications 1 à 27, **caractérisé en ce que** le deuxième élément de liaison (2) est traversé par un alésage coaxial par rapport à son axe longitudinal (17), de sorte qu'un tournevis peut être passé dans cet alésage.

29. Dispositif selon l'une des revendications 1 à 28, **caractérisé en ce que** des pointes (30) qui peuvent être reliées aux fragments d'os, aux corps de vertèbre ou à des parties d'implant sont ménagées sur les éléments de liaison (1; 2).

30. Dispositif selon l'une des revendications 1 à 28, **caractérisé en ce que** des lames (31) qui peuvent être amenées à se placer contre des fragments d'os, des corps de vertèbre ou des parties d'implant sont installées sur les éléments de liaison (1; 2).

31. Dispositif selon l'une des revendications 1 à 28, **caractérisé en ce que** des plaques (32) en forme de U qui peuvent être amenées à se placer contre les parties de l'implant sont prévues sur les éléments de liaison (1; 2).

32. Dispositif selon l'une des revendications 1 à 28, **caractérisé en ce que**
A) la liaison entre les éléments de liaison (1; 2) et les implants reliés fixement aux corps de vertèbre ou aux fragments d'os, par exemple des vis à os, sont configurés en forme d'articulation sphérique et
B) **en ce que** la liaison en forme d'articulation sphérique peut être bloquée angulairement de manière réversible.
